# EUROPÄISCHE PATENTSCHRIFT

(11) **EP 0 842 183 B1**
(45) Veröffentlichungstag und Bekanntmachung des Hinweises auf die Patenterteilung: **17.10.2001**
(21) Anmeldenummer: 96927594.0
(22) Anmeldetag: 24.07.1996
(51) Int. Cl.: C07F 15/00, B01J 31/16, C07B 37/04, C07C 253/30

(54) **KATALYSATOREN ZUR DURCHFÜHRUNG VON KREUZKUPPLUNGSREAKTIONEN**
CATALYST FOR CROSS-COUPLING REACTIONS
CATALYSEURS DESTINES A LA MISE EN UVRE DE REACTIONS DE COUPLAGE CROISE

(30) Priorität: 25.07.1995 DE 19527118; 25.09.1995 DE 19535528; 17.05.1996 DE 19620023
(43) Veröffentlichungstag der Anmeldung: 20.05.1998
(73) Patentinhaber: Clariant GmbH, 65929 Frankfurt am Main (DE)
(72) Erfinder: HABER, Steffen, D-61462 Königstein (DE); EGGER, Norbert, D-65185 Wiesbaden (DE)
(86) Internationale Anmeldenummer: EP9603266
(87) Internationale Veröffentlichungsnummer: WO9705151

(56) Entgegenhaltungen:
- EP-A- 0 679 619
- EP-A- 0 694 530
- FR-A- 2 693 188
- TETRAHEDRON LETTERS, Bd. 36, Nr. 9, 1995, Seiten 1443-1446, XP002016760 GENET, J.P. ET AL.: "SUZUKI-TYPE CROSS COUPLING REACTIONS USING PALLADIUM-WATER SOLUBLE CATALYST. SYNTHESIS OF FUNCTIONALIZED DIENES"
- CHEMICAL ABSTRACTS, vol. 111, no. 9, 28.August 1989 Columbus, Ohio, US; abstract no. 78233h, OKAMOTO, Y. ET AL.: "CROSS-COUPLING OF BROMONITROBENZENES WITH ORGANOZINC REAGENTS IN MIXED SOLVENTS" XP002016754 & CHEM. EXPRESS, Bd. 4, Nr. 3, 1989, Seiten 181-184,

## Beschreibung

Die Erfindung betrifft einen Palladiumkatalysator mit einem wasserlöslichen Phosphanliganden, vorzugsweise zur Durchführung von Kreuzkupplungsreaktionen, ein Verfahren zu dessen Herstellung sowie dessen Verwendung in Kreuzkupplungsreaktionen.

Kreuzkupplungsreaktionen von aromatischen Borverbindungen, wie Boronsäuren, und aromatischen Halogenverbindungen oder Perfluoralkylsulfonaten werden seit einigen Jahren in steigendem Umfang zum Aufbau mehrkerniger aromatischer Systeme benutzt. Beispielsweise dienen solche Verfahren zur Herstellung von pharmazeutischen Wirkstoffen und Komponenten von Flüssigkristallmischungen.

Die üblicherweise verwendeten Katalysatoren, wie Pd[P(Ph₃)]₄, PdCl₂(PPh₃)₂ (Chem. Express 1989, 4 (3), 181-184) oder PdCl₂(4PPh₃)4NaBH₄, liefern aber nur mit Brom oder lodaromaten die Kupplungsprodukte in nennenswerten Ausbeuten. Die hohen Kosten dieser Ausgangsverbindungen erschweren eine wirtschaftliche Überführung der Prozesse in einen größeren Produktionsmaßstab.

In der EP-A 0 372 313 sind Palladiumkatalysatoren mit wasserlöslichen Phosphanliganden offenbart, die beispielsweise bei der Kreuzkupplungsreaktion von Alkinen mit Allenen Verwendung finden.

Die beschriebenen Systeme enthalten stets Palladium in der Oxidationstufe (O).

In der EP-A 0 694 530 ist ein Verfahren zur Herstellung mehrkerniger aromatischer Verbindungen durch Kreuzkupplung von aromatischen Borverbindungen mit aromatischen Halogenverbindungen oder - Perfluoralkylsulfonaten unter Palladiumkatalyse in Gegenwart mindestens eines wasserlöslichen Komplexliganden beschrieben, welches dadurch gekennzeichnet ist, daß das Reaktionsmedium eine wäßrige und eine organische Phase enthält und das Palladium in Form einer in der organischen Phase löslichen Palladiumverbindung zugesetzt wird.

Obwohl nach diesem Verfahren bereits sehr gute Ergebnisse erzielt werden, bleibt doch Raum für Verbesserungen, insbesondere was die Verwendung von wohlfeilen Chloraromaten als Ausgangsmaterial betrifft.

Es wurde nun überraschend gefunden, daß sich Palladiumkatalysatoren mit besonders hoher Aktivität durch Reaktion von Palladium(ll)verbindungen, wasserlöslichen Phosphanliganden und einem Sulfoxid oder einem mehrwertigen Alkohol herstellen lassen.

Ein Gegenstand der vorliegenden Erfindung ist daher ein Katalysatorsystem, insbesondere zur Durchführung von Kreuzkupplungsreaktionen, erhältlich durch Umsetzung
a) einer Palladium(ll)verbindung mit
b) einem wasserlöslichen Phosphanliganden und
c) einem Sulfoxid oder mehrwertigem wasserlöslichen Alkohol gemäß Anspruch 1.

Ein weiterer Gegenstand der Erfindung ist ein Verfahren zur Herstellung eines Katalysatorsystems, insbesondere zur Durchführung von Kreuzkupplungsreaktionen, dadurch gekennzeichnet, daß man
a) eine Palladium(ll)verbindung mit
b) einem wasserlöslichen Phosphanliganden und
c) einem Sulfoxid oder mehrwertigem wasserlöslichen Alkohol gemäß Anspruch 1
umsetzt.

Ebenfalls Gegenstand der Erfindung ist die Verwendung eines Katalysatorsystems, erhältlich durch Umsetzung
a) einer Palladium(ll)verbindung mit
b) einem wasserlöslichen Phosphanliganden und
c) einem Sulfoxid oder mehrwertigem Alkohol,
zur Durchführung von Kreuzkupplungsreaktionen.

Erfindungsgemäße Katalysatorsysteme zeichnen sich durch eine besonders hohe Aktivität und die Möglichkeit der mehrfachen Verwendung aus. Sie sind insbesondere auch für Kupplungsreaktionen geeignet, bei denen Chloraromaten als Ausgangssstoffe verwendet werden.

Geeignet als Komponente a sind Palladium(II)verbindungen, vorzugsweise Palladium(ll)salze, Palladiumtetrachlorosäure oder deren Salze, vorzugsweise Alkalimetallsalze. Bevorzugte Verbindungen sind beispielsweise Palladiumacetylacetonate, Palladiumhalogenide, Allylpalladiumhalogenide und Palladiumbiscarboxylate, besonders bevorzugt und Palladiumacetylacetonate, Palladium(ll)halogenide, Palladiumtetrachlorosäure und deren Salze.

Ganz besonders geeignet als Komponente a sind Pd(II)Cl₂/3 NaOAc, Pd(ac)₂, K₂PdCl₄, Na₂PdCl₄, K₂Pd₂Cl₆ und Na₂Pd₂Cl₆ und H₂PdCl₄.

Es können natürlich auch Gemische von zwei oder mehreren Palladiumverbindungen als Komponente a eingesetzt werden.

In einer bevorzugte Ausgestaltung der Erfindung enthält das Katalysatorsystem einen oder mehrere Zusatzstoffe, wie Natriumacetat, die als Lösungsvermittler für die Palladiumverbindung in dem System Sulfoxid bzw. mehrwertiger Alkohol und gegebenenfalls Wasser dient. Besonders bevorzugt wird Natriumacetat, insbesondere in einem molaren Verhältnis von 1 bis 4, bevorzugt 3, bezogen auf die Palladiumverbindung, eingesetzt.

Als wasserlösliche Phosphanliganden eignen sich vorzugsweise mit Carboxylat-bzw. Carbonsäure, Ammonium, Phosphonium, Sulfonat- bzw. Sulfonsäure, Phosphonat- bzw. Phosphonsäure, Polyalkohole mit geeigneter Anzahl von Hydroxyfunktionen versehene Gruppen, Polyalkylenglykole mit geeigneter Kettenlänge, versehene Tri-n-alkylphosphane, Triarylphosphane, Dialkylarylphosphane, Alkyldiarylphosphane und Heteroarylphosphane wobei die drei Substituenten am Phosphor gleich oder verschieden, chiral oder achiral sein können und wobei einer oder mehrere der Substituenten die Phosphorgruppen mehrerer Phosphane verknüpfen können und wobei ein Teil dieser Verknüpfung auch ein oder mehrere Metallatome sein können.

Besonders bevorzugt sind wasserlösliche Phosphane, die mindestens eine Arylgruppe am Phosphor enthalten, d.h. Triarylphosphane, Diarylalkylphosphane und Dialkylarylphosphane.

Besonders bevorzugt eingesetzte wasserlösliche Phosphane sind solche der allgemeinen Formeln (I) bis (VII), 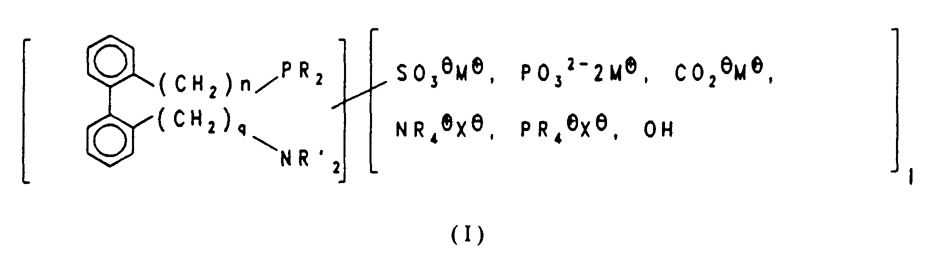 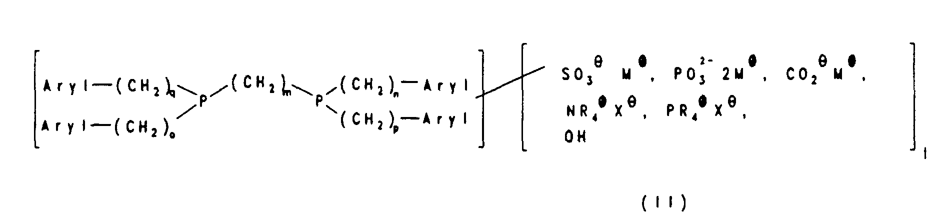 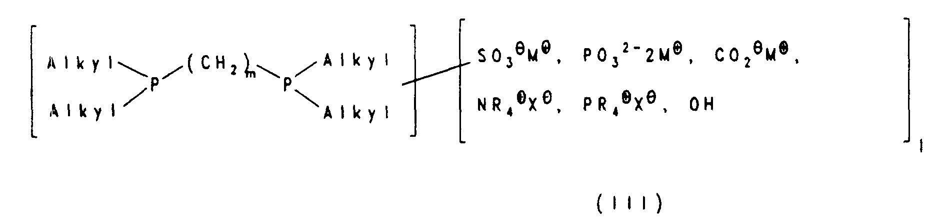 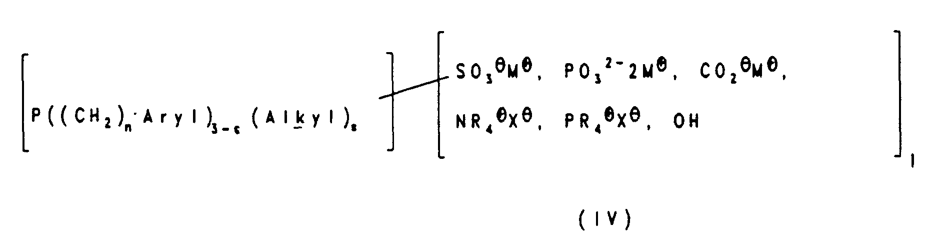 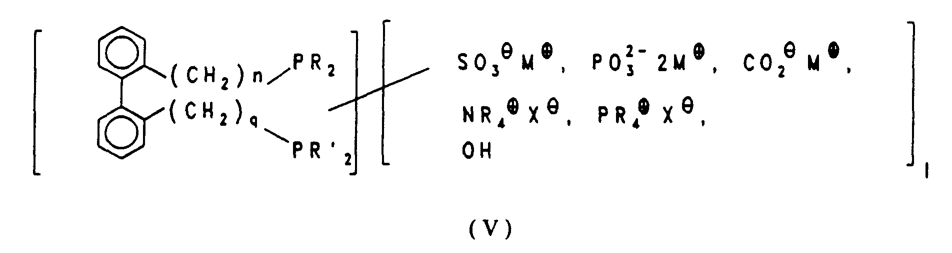 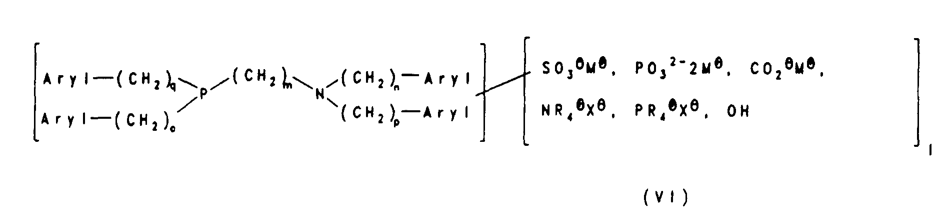 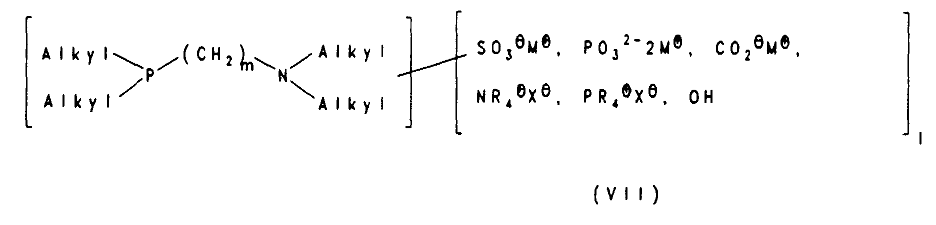 wobei die Symbole und Indizes folgende Bedeutungen haben:
- Aryl:: eine Phenyl- oder Naphthylgruppe, die auch einen oder mehrere Substituenten R tragen kann;
- Alkyl:: eine geradkettige oder verzweigte Alkylgruppe mit 1 bis 8 Kohlenstoffatomen;
- R,R':: Alkyl, Aryl oder Aralkyl mit 1 bis 18 Kohlenstoffatomen;
- M:: Alkali-, Erdalkalimetall oder NR₄;
- X:: Halogen, BF₄, OSO₂CF₃, 1/2[SO₄];
- l,m:: 1 bis 8;
- n,o,p,q:: 0, 1 bis 8;
- s:: 0, 1 bis 3.

Im folgenden sind Beispiele für besonders bevorzugte wasserlösliche Komplexliganden aufgeführt:
(R hat dabei, wenn nicht anders vermerkt, die in den Formeln (I) bis (VII) angegebene Bedeutung)

### 1. Sulfonierte Phosphane

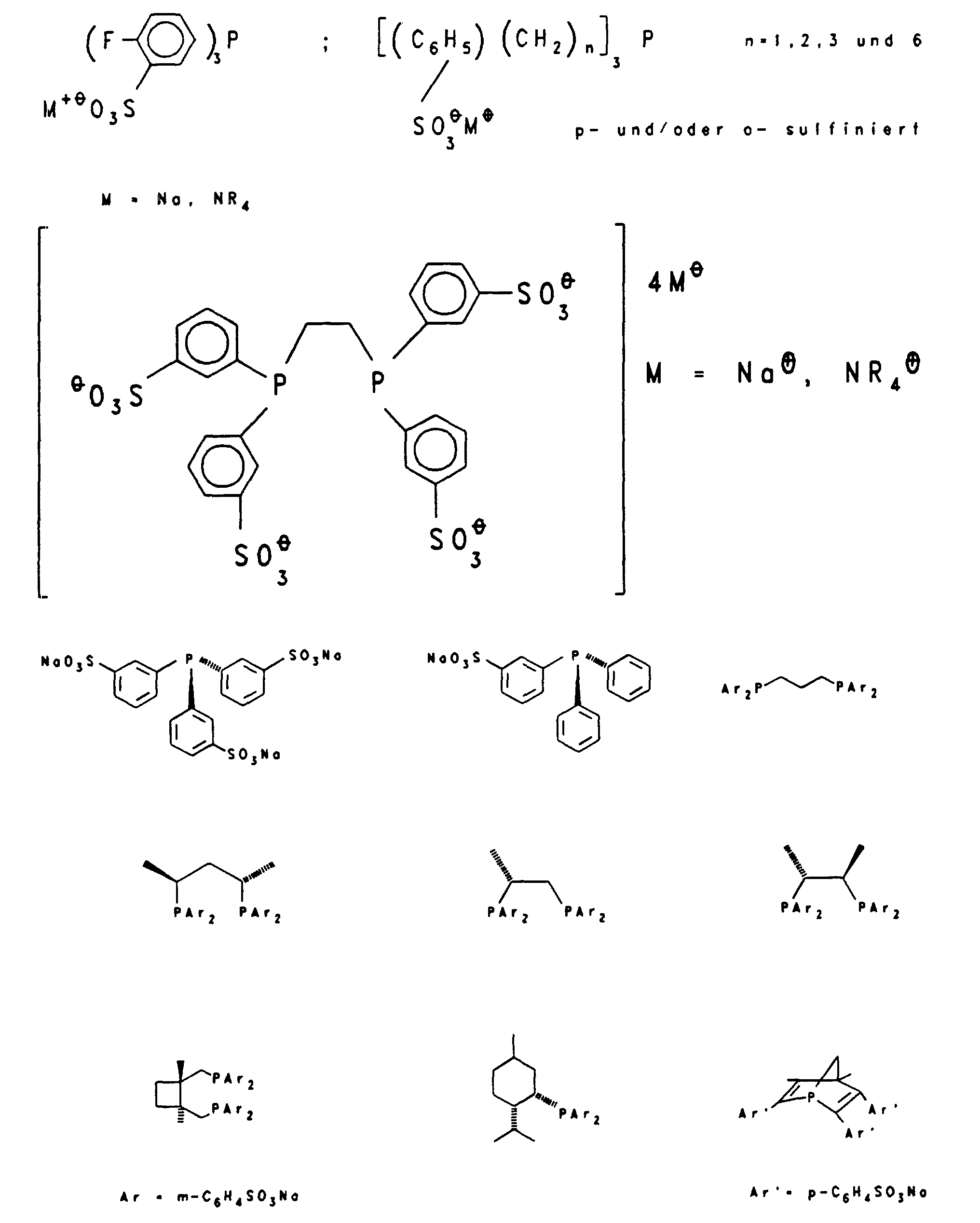

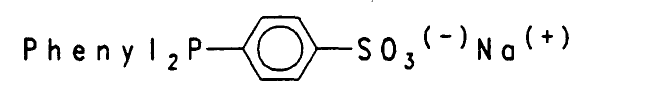

R₃₋ₙP(p-C₆H₄SO₃K)ₙ R = C₆H₅, 2-Pyridyl, 3-Pyridyl; n = 1-3
P[p-OC₆H₄SO₃(NH(i-octyl)₃]₃

### 1.1 Phosphane mit hydrophilen Gruppen in der Peripherie

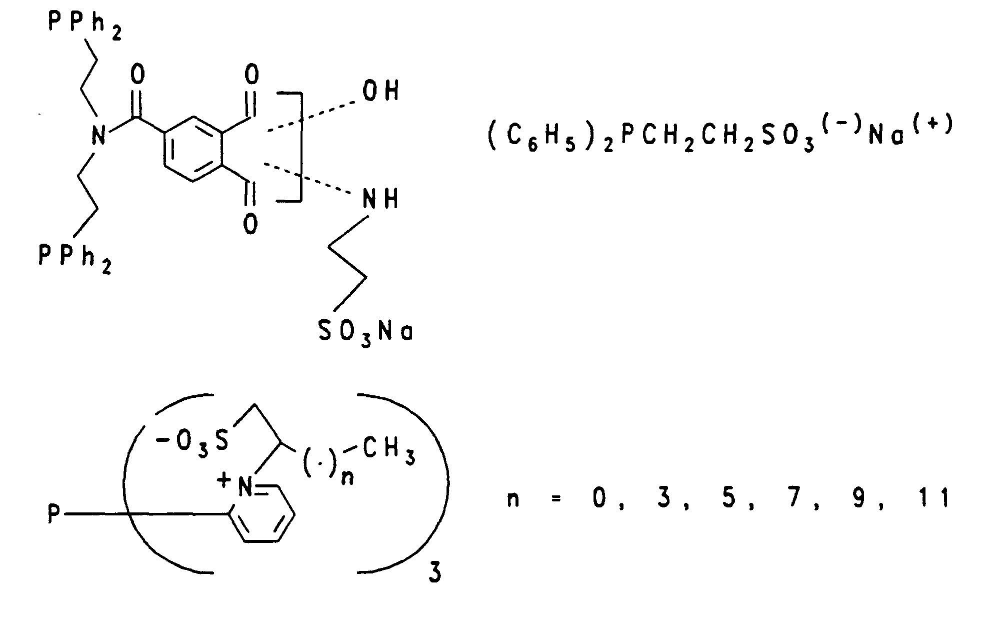

### 2. Phosphane mit quaternisierten Aminoalkyl- und Aminoaryl-Substituenten

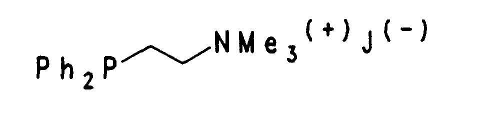

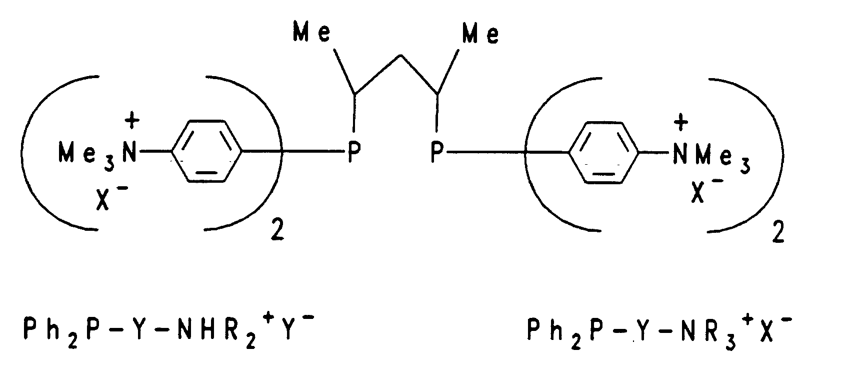

Y = -CH₂CH₂-, -CH(CH₃)CH₂-, -CH₂CH(CH₃)CH₂-; R = CH₃; X = J^{⊖}, Bu^{⊖}, Cl^{⊖}, OSO₂CF₃^{⊖}, BF₄^{⊖}

### 3. Carboxylierte Phosphane

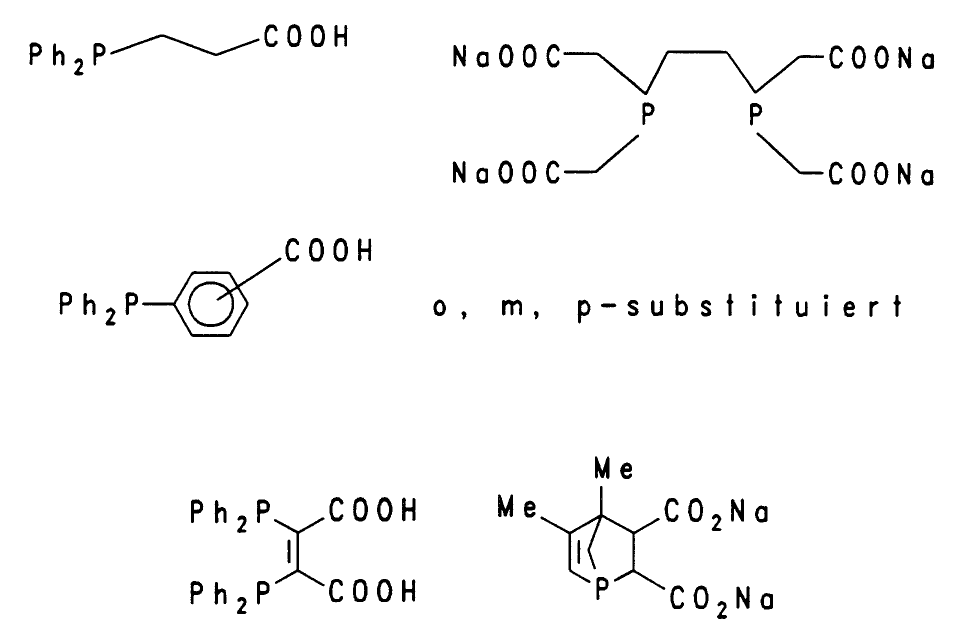

Insbesondere bevorzugt sind:

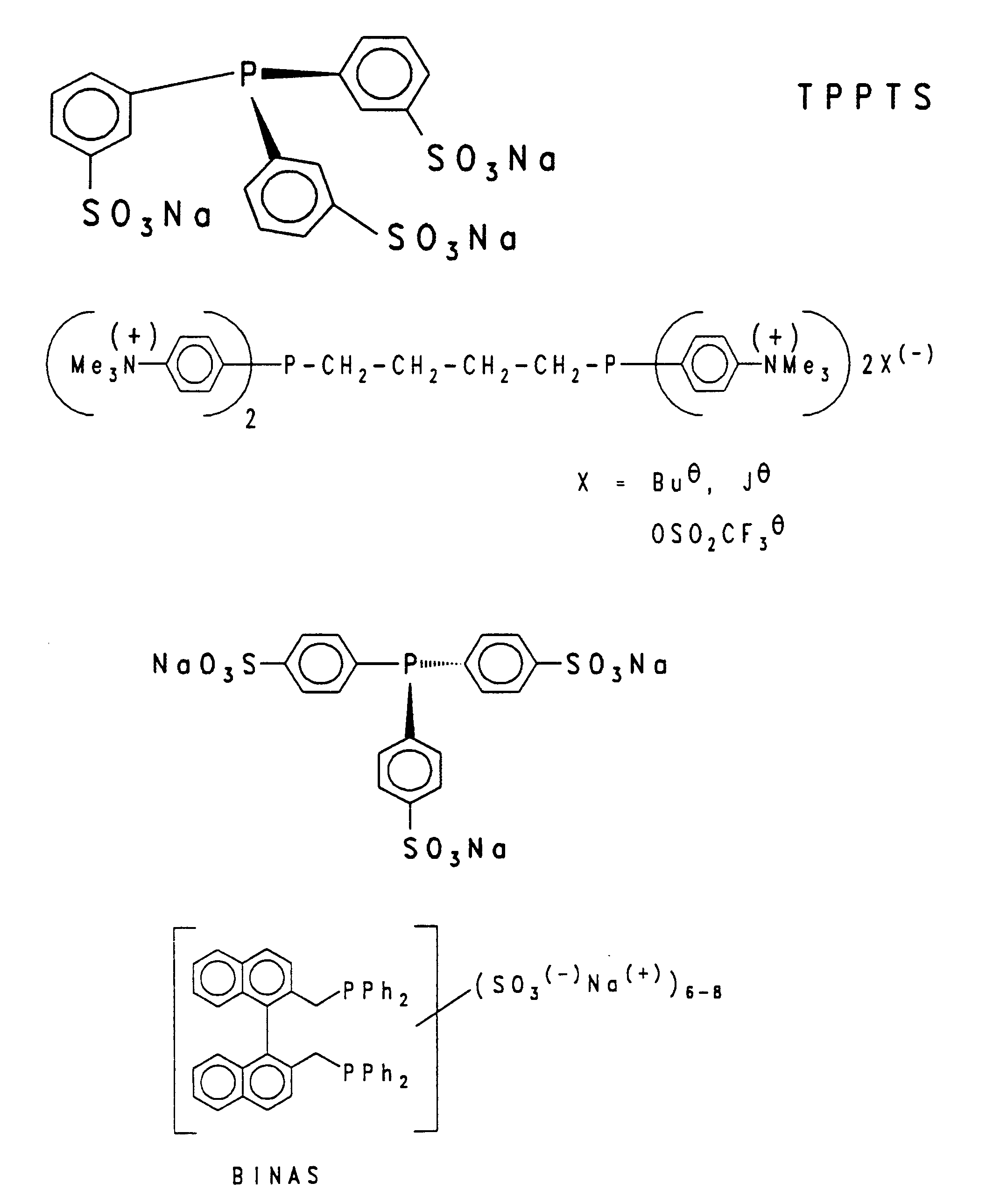

Es können natürlich auch mehrere phosphorhaltige Liganden eingesetzt werden.

Die erfindungsgemäß eingesetzten phosphorhaltigen Liganden sind an sich bekannt. Teilweise sind es kommerzielle Produkte oder sie sind mit ihrer Synthese beispielsweise in Houben-Weyl, Methoden der Organischen Chemie, Georg-Thieme-Verlag, Stuttgart, beschrieben.

Wasserlösliche Liganden können beispielsweise nach W.A. Herrmann und C.W. Kohlpainter, Angew. Chem. Int. Ed. Engl. 1993, 32, 1524 oder der dort zitierten Literatur hergestellt werden. Die Herstellung von BINAS ist in der EP-A 0 571 819 bzw. US-A 5,347,045 beschrieben. Ein wäßrige 0,6 molare Lösung des Trinatriumsalzes von TPPTS ist kommerziell erhältlich (Hoechst AG, Deutschland).

Der phosphorhaltige Ligand wird erfindungsgemäß in einem Verhältnis von 1 bis 20 Phosphoräquivalenten, bevorzugt 2 bis 12, besonders bevorzugt 2 bis 6, ganz besonders bevorzugt 4, bezogen auf die Pd-Verbindung, eingesetzt.

Bevorzugte mehrwertige Alkohole sind solche, die wasserlöslich sind.
Besonders bevorzugt sind Glykole, Glycerin, Oligoglyceride, die auch teilverestert sein können, Di-, Tri- und Tetraethylenglykol oder auch Polyethylenglykole der allgemeinen Formel (VIII), 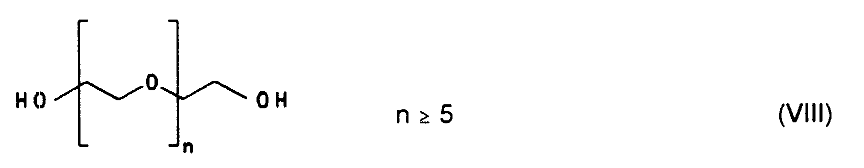 mehrwertige Alkan- oder Alkenole, wie 1,4-Butandiol, 1,3-Propandiol, 1,2-Propandiol, Pentaerythrit, 2-Ethylhexan-1,3-diol, 2-(Hydroxymethyl)-2-methyl-1,3-propandiol, 2-Methyl-2,4-pentandiol, 1,4-cis-Butendiol, mehrwertige Cycloalkanole, wie Cyclohexandiol, mehrwertige, Arylgruppen enthaltende Alkanole, wie 1-Phenyl-1,2-ethandiol, mehrwertige Aminoalkohole, wie Diethanolamin, Triethanolamin, 2-Annino- 2-methyl-1,3-propandiol, 3-(Aminomethyl)-1,2-propandiol, 3-Amino-1,2-propandiol, 2-Amino-1,3-propandiol-oxalat, 3-(Diethylamino)-1,2-propandiol, Ethylendiamin-N,N,N',N'-tetra-2-propandiol, mehrwertige Iminoalkohole, wie N-Butyl- und N-tert.-Butyl-2,2'-iminodiethanol, 1,1'-Iminodi-3-propanol, N-Methyl-2,2'-iminodiethanol, N-Phenyl-2,2'-iminodiethanol, oder auch Verbindungen wie 1,1',1"-Nitrilo-tri-2-propanol, 1,3,5-Tri-(2-hydroxyethyl)-isocyanursäure und Dihydroxyaceton.

Ganz besonders bevorzugt sind Glykol, Glycerin, 1,4-Butandiol, 1,2-Propandiol, Triethylenglykol, Diethylenglykol, Diethanolamin und Triethanolamin und davon insbesondere Glykol, Glycerin, 1,4-Butandiol und 1,2-Propandiol.

Es können natürlich auch mehrere mehrwertige Alkohole eingesetzt werden. Bevorzugte Sulfoxide sind solche der allgemeinen Formel (IX): 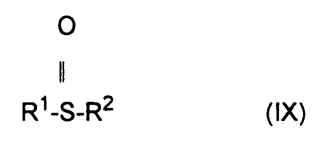 R¹, R² sind aliphatische oder aromatische Kohlenwasserstoffe, die gegebenenfalls substituiert oder miteinander verknüpft sein können.

Besonders bevorzugte Sulfoxide sind Dimethylsulfoxid (DMSO), Diphenylsulfoxid, Methylphenylsulfoxid und Dibenzylsulfoxid.

Bevorzugt werden wasserlösliche Sulfoxide eingesetzt. Besonders bevorzugte wasserlösliche, Sulfoxide ist DMSO.

Es können natürlich auch mehrere Sulfoxide oder deren Gemische, gegebenenfalls auch mit mehrwertigen Alkoholen, eingesetzt werden.

Die mehrwertigen, wasserlöslichen Alkohole bzw. das Sulfoxid werden vorzugsweise in einem Gewichtsverhältnis von 0,1 bis 10 000, bezogen auf die Palladium(II)verbindung zugesetzt.

Bevorzugt enthält das erfindungsgemäße Katalysatorsystem Wasser, beispielsweise durch Zugabe des Phosphanliganden als wäßrige Lösung.

Die Herstellung des erfindungsgemäßen Katalysatorsystems kann nach verschiedenen Varianten erfolgen.

Die Palladiumverbindung kann beispielsweise durch Lösen in dem Sulfoxid oder dem mehrwertigen Alkohol unter Zuhilfenahme von Zusatzstoffen wie Natriumacetat zuerst gelöst werden und anschließend mit dem in Wasser gelösten Phosphanliganden, zur Bildung des erfindungsgemäßen Katalysatorsystems, zur Reaktion gebracht werden. Das Katalysatorsystem entsteht aber auch beim gleichzeitigen Mischen der Einzelkomponenten.

Zur Verwendung des erfindungsgemäßen Katalysatorsystems ist es bevorzugt die Palladium(ll)Verbindung in einem mehrwertigen Alkohol oder Sulfoxid, vorzugsweise DMSO oder Glykol zu lösen, mit dem wasserlöslichen Phosphanliganden oder einer Lösung desselben zu versetzen und die so gebildete Katalysatorlösung zu den übrigen Reaktanden zu geben.

Ebenso bevorzugt ist es Palladium oder eine Palladium-Verbindung in einem mehrwertigen Alkohol, Sulfoxid, vorzugsweise DMSO oder Glykol, zu lösen, diese Lösung mit den übrigen Reaktanden zu versetzen und anschließend den wasserlöslichen Phosphanliganden oder eine Lösung desselben zuzugeben.

Weiterhin bevorzugt ist es die Palladium(ll)verbindung, beispielsweise in wäßriger Lösung vorverlegen, mit dem wasserlöslichen Phosphanliganden, gegebenenfalls als Lösung, zu versetzen und diese Lösung zu einer Mischung der Edukte, gegebenenfalls eines Lösungsmittels und des mehrwertigen Alkohols oder Sulfoxids zu geben.

Vorzugsweise weisen erfindungsgemäße Katalysatorsysteme im ³¹P-NMR Spektrum breite Signale von 36 bis 32 ppm und 8 bis 4 ppm auf (bei virtueller Referenzierung extern auf 85 % Phosphorsäure, Spektrometer Bruker DRX 400).

Die erfindungsgemäßen Katalysatorsysteme zeigen beispielsweise in den folgenden Reaktionen katalytische Aktivität: Telomerisierungen, Addition CH-acider Verbindugnen an Butadien, Hydrierungen, Reaktion von Nitroverbindungen, vorzugsweise Nitroaromaten.

Sie finden daher bei solchen Reaktionen Verwendung als Katalysator.

Bevorzugt ist die Verwendung als Katalysator für Kohlenstoff-Kohlenstoff-Verknüpfungsreaktionen, insbesondere für die Kreuzkupplungsreaktion von Boronsäuren mit, vorzugsweise aromatischen, Halogenverbindungen, insbesondere Chloraromaten.

Im folgenden wird beispielhaft die Verwendung erfindungsgemäße Katalysatorsysteme für Kreuzkupplungsreaktionen von aromatischen Boronsäuren mit Halogenaromaten beschrieben.

Diese Reaktion ist dadurch gekennzeichnet, daß man
a) eine aromatische Borverbindung mit
b) einer aromatischen Halogenverbindung oder einem aromatischen Perfluoralkylsulfonat in Gegenwart
c) einer Base, und
d) einem erfindungsgemäßen Katalysatorsystem umsetzt.

Das erfindungsgemäße Katalysatorsystem wird bei dem Verfahren mit einem Anteil von 0,001 bis 10 Mol-%, bevorzugt 0,01 bis 5 Mol-%, besonders bevorzugt 0,05 bis 3 Mol-%, insbesondere bevorzugt 0,05 bis 1,5 Mol-%, bezogen auf die aromatische Halogenverbindung oder das aromatische Perfluoralkylsulfonat, eingesetzt.

Basen, die bei dem Verfahren üblicherweise Verwendung finden, sind Alkalimetallfluoride, Alkali- und Erdalkalimetallhydroxide, Alkali- und Erdalkalimetallcarbonate, Alkalimetallhydrogencarbonate, Alkali- und Erdalkalimetallacetate, Alkali- und Erdalkalimetallalkoholate, sowie primäre, sekundäre und tertiäre Amine.

Besonders bevorzugt sind Alkalimetallfluoride, Alkali- und Erdalkalimetallhydroxide, Alkali- und Erdalkalimetallcarbonate und Alkalimetallhydrogencarbonate. Insbesondere bevorzugt sind Alkalimetallfluoride, wie Kaliumfluorid und Caisiumfluorid, Alkalimetallhydroxide, wie Natriumhydroxid und Kaliumhydroxid, sowie Alkalimetallcarbonate und Alkalimetallhydrogencarbonate, wie Lithiumcarbonat, Natriumcarbonat und Kaliumcarbonat.

Bei Verwendung fester Basen, wie Na₂CO₃, wird der mehrwertige Alkohol oder das Sulfoxid vorzugsweise in größeren Mengen oder als Lösungsmittel eingesetzt, um eine geeignete Suspendierung der Base und damit ein rührfähiges Gemisch zu erreichen.

Es können natürlich auch mehrere Basen zugesetzt werden.

Die Base wird bevorzugt mit einem Anteil von 100 bis 1000 Mol-%, besonders bevorzugt 100 bis 500 Mol-%, ganz besonders bevorzugt 100 bis 400 Mol-%, insbesondere 100 bis 290 Mol-%, bezogen auf die aromatische Borverbindung, eingesetzt.

Bevorzugte Ausgangsverbindungen sind zum einen aromatische Borverbindungen der Formel (Xl),

Aryl ― BQ₁Q₂ (XI)

worin
- Aryl: ein aromatischer Rest ist und
- Q₁, Q₂: gleich oder verschieden -OH, C₁-C₄-Alkoxy, C₁-C₄-Alkyl, Phenyl, das gegebenenfalls durch C₁-C₄-Alkyl, C₁-C₄-Alkoxy oder Halogen substituiert sein kann, oder Halogen bedeuten oder Q₁ und Q₂ zusammen bilden eine C₁-C₄-Alkylendioxy-Gruppe, eine Methylengruppe, die gegebenenfalls durch eine oder zwei C₁-C₄-Alkylgruppen substituiert sein kann, oder Q₁ und Q₂ und das Boratom zusammen sind Teil eines Boroxinrings der Formel (Xl): 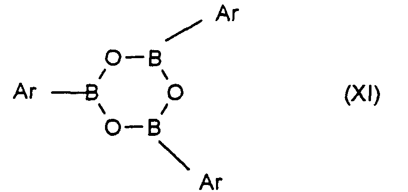
- Aryl: bedeutet bevorzugt einen Phenyl-, Naphthyl-, Pyrimidyl-, Pyridin-, Pyrazin-, Pyradazin-, 1,3-Thiazol, 1,3,4-thiadiazol- oder Thiophenylrest, die alle gegebenenfalls substituiert sein können, beispielsweise mit Halogen, Cyano, Alkyl oder Alkoxygruppen.
- Q₁, Q₂: sind vorzugsweise gleich oder verschieden -OH, C₁-C₄-Alkoxy oder Halogen oder Q₁ und Q₂ zusammen bilden eine C₁-C₄-Alkylendioxy-Gruppe oder Q₁ und Q₂ und das Boratom zusammen sind teil eines Boroxinrings der Formel (Xl):

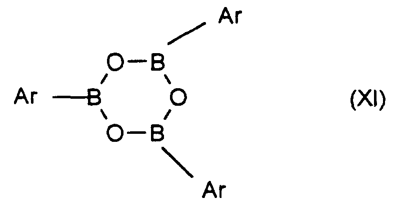

Besonders bevorzugt bedeutet Aryl eine unsubstituierte oder substituierte Phenyl- oder Naphthylgruppe.

Die verwendeten aromatischen Borverbindungen sind entweder bekannt oder können nach an sich bekannten Methoden, wie beispielsweise in Houben Weyl Methoden der Organischen Chemie, Georg Thieme-Verlag, Stuttgart, Band 13/3a beschrieben, hergestellt werden. So ist es beispielsweise möglich, aus aromatischen Alkalimetall- und Magnesiumverbindungen durch Umsetzung mit Trialkoxyboranen und anschließender Hydrolyse Boronsäuren zu erhalten.

Die zweite Klasse von Ausgangsverbindungen Verfahren sind aromatische Verbindungen der Formel (XII)

Aryl - X (XII)

wobei
- Aryl: einen aromatischen Rest und
- X: Cl, Br, l oder ein Perfluoralkylsulfonat bedeutet.
- X: bedeutet vorzugsweise Cl.
- Aryl: bedeutet vorzugsweise einen unsubstituierten oder substitueirten Phenyl-, Naphthyl-, Pyridin-, Pyrimidin-, Pyrazin-, Pyridazin-, 1,3-Thiazol-, 1,3,4-Thiadiazol- oder Thiophenrest, wobei der oder die Substituenten beispielsweise Halogen, CN, Alkyl-, Alkoxy- oder weitere Arylgruppen sind.

Die verwendeten aromatischen Halogenverbindungen und Perfluoralkylsulfonate sind entweder bekannt oder können nach bekannten Methoden, wie beispielsweise in Houben Weyl, Methoden der Organischen Chemie,
Georg Thieme Verlag, Stuttgart, Band 5/3 und 5/4 beschrieben, hergestellt werden. Beispielsweise lassen sich aromatische Halogenide dadurch erhalten, daß man in einem entsprechenden Diazoniumsalz die Diazoniumgruppe durch Chlor, Brom oder lod ersetzt.

Des weiteren lassen sich Hydroxy-Stickstoffheterocyclen mit Hilfe von Phosphortrihalogeniden und Phosphoroxytrihalogeniden in die entsprechenden Halogenide überführen.

Zur Durchführung des Verfahrens werden die Edukte, die Base und das erfindungsgemäße Katalysatorsystem nach den oben angegebenen Varianten gemischt und bei einer Temperatur von 0 bis 200°C, bevorzugt 30 bis 170°C, besonders bevorzugt 50 bis 150°C, über einen Zeitraum von 1 bis 100 h, vorzugsweise 5 bis 70 h, besonders bevorzugt 5 bis 50 h umgesetzt.

Die Aufarbeitung erfolgt nach bekannten, dem Fachmann geläufigen Methoden. Beispielsweise kann das Produkt durch Extraktion oder Ausfällen vom Reaktionsgemisch abgetrennt und anschließend nach dem jeweiligen Produkt angemessenen Methoden, wie Umkristallisation, Destillation, Sublimation, Zonenschmelzen, Schmelzkristallisation oder Chromatographie, weiter aufgereinigt werden.

Die so hergestellten Verbindungen eignen sich zum Einsatz als flüssigkristalline Materialien oder können als Zwischenprodukte für die Herstellung weiterer flüssigkristalliner Verbindungen verwendet werden. Des weiteren werden sie als Vorprodukte für Pharmazeutika, Kosmetika, Fungizide, Herbizide, Insektizide, Farbstoffe, Detergenzien und Polymere, einschließlich von Zusatzstoffen derselben, eingesetzt.

Die Erfindung wird durch die Beispiele näher erläutert, ohne sie dadurch beschränken zu wollen.

### Beispiele:

### Beispiel 1

0,388 g Palladium(ll)chlorid und 0,54 g Natriumacetat werden in 24 ml DMSO gelöst. Man rührt 30 Minuten bei Raumtemperatur. Anschließend versetzt man mit 14,6 ml TPPTS/H₂O-Lösung (0,6 mol/l) und rührt 30 Minuten nach.

### Beispiel 2

0,388 g Palladium(ll)chlorid und 0,54 g Natriumacetat werden in 24 ml Ethylenglykol gelöst. Man rührt 30 Minuten bei Raumtemperatur nach. Anschließend versetzt man mit 14,6 ml TPPTS/H₂O-Lösung (0,6 mol/l) und rührt 30 Minuten nach.

### Beispiel 3

1,069 g Tetrachloropalladiumsäure (20 Gew.-% Palladium in Wasser) werden mit 24 ml Wasser verdünnt und anschließend mit 14,6 ml einer 0,6 molaren TPPTS/H₂O-Lösung versetzt. Man rührt 30 Minuten nach. Anschließend gibt man 50 ml Ethylenglykol zu.

Die Lösungen zeigen direkt nach dem Ansetzen im ³¹P-NMR-Spektrum breite Signale von 36 bis 32 ppm und 8 bis 4 ppm. Die Referenzierung erfolgte virtuell, extern auf 85 % Phosphorsäure. Spektrometer war ein DRX 400 der Firma Bruker.

### Anwendungsbeispiele

Die bei den Katalysatorlösungen angegebenen Molprozente betreffen den Pd(lll)-Gehalt der Katalysatorlösung und beziehen sich auf die Halogenverbindung

### Anwendungsbeispiel 1

15g 2-Chlorbenzonitril, 14,8 g p-Toluolboronsäure und 28,9 g Natriumcarbonat wurden in 40 ml Glykol und 10 ml Wasser auf 120°C erhitzt. Bei 80°C gab man 0,1 Mol% einer nach Beispiel 1 hergestellten Katalysatorlösung zu. Nach beendeter Reaktion wurde mit 50 ml Xylol versetzt und die organische Phase abgetrennt. Durch Destillation erhielt man 19 g 2-Cyano-4'-methyl-biphenyl (Sdp. 140°C/mbar).

### Vergleichsversuch

15 g 2-Chlorbenzonitril, 14,8 g p-Toluolboronsäure und 28,9 g Natriumcarbonat wurden in 40 ml Glykol und 10 ml Wasser auf 120 °C erhitzt. Bei 80°C gab man 0,1 Mol-% Pd(O)(TPPTS)₃. 9H₂O gelöst in 3 ml H₂O zu. Die Reaktionsmischung wurde 12 h bei 120°C gehalten. Nach Abkühlen wurde mit 50 ml Xylol versetzt und die organische Phase abgetrennt. Durch Destillation erhielt man 10,5 g 2-Cyano-4'-methyl-biphenyl (Sdp. 140°C/mbar).

### Anwendungsbeispiel 2

15 g 2-Chlorbenzonitril, 14,8 g p-Toluolboronsäure und 28,9 g Natriumcarbonat wurden in 40 ml Glykol und 10 ml Wasser auf 120°C erhitzt. Bei 80°C gab man 0,1 Mol% einer nach Beispiel 2 hergestellten Katalysatorlösung zu. Nach beendeter Reaktion wurde mit 50 ml Xylol versetzt und die organische Phase abgetrennt. Durch Destillation erhielt man 18,5 g 2-Cyano-4'-methyl-biphenyl (Sdp. 140°C/mbar).

### Anwendungsbeispiel 3

### Vorbereitung der Katalysatorlösung:

0,388 g Palladium(ll)chlorid und 14,6 ml TPPTS/H₂O-Lösung (0,6 mol/l) wurden 60 Minunten bei Raumtemperatur gerührt. Man erhielt eine gelbe Reaktionslösung von

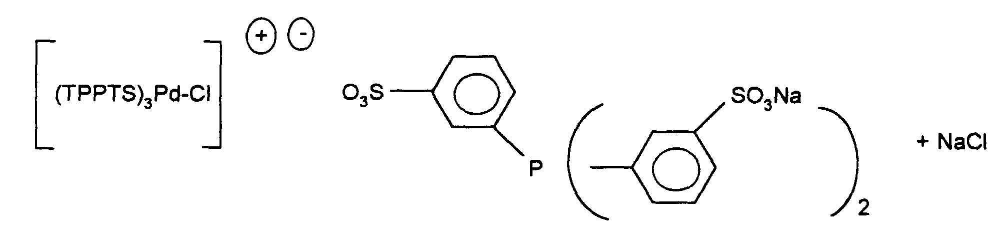

15 g 2-Chlorbenzonitril, 14,8 g p-Toluolboronsäure und 28,9 g Natriumcarbonat wurden in 40 ml Glykol und 10 ml Wasser auf 120°C erhitzt. Bei 80°C gab man 0,1 Mol% einer oben beschriebenen Katalysatorlösung zu. Nach beendeter Reaktion wurde mit 50 ml Xylol versetzt und die organische Phase abgetrennt. Durch Destillation erhielt man 18,7 g 2-Cyano-4'-methyl-biphenyl (Sdp. 140°C/mbar).

### Anwendungsbeispiel 4

### Vorbereitung der Katalystarlösung:

0,388 g Palladium(II)chlorid und 0,33 g Kaliumchlorid wurden in 10 ml Wasser gelöst. Anschließend versetzte man mit 14,6 ml TPPTS/H₂O-Lösung (0,6 mol/l).

15 g 2-Chlorbenzonitril, 14,8 g p-Toluolboronsäure und 28,9 g Natriumcarbonat wurden in 40 ml Glykol und 10 ml Wasser auf 120°C erhitzt. Bei 80°C gab man 0,1 Mol% einer oben beschriebenen Katalysatorlösung zu. Nach beendeter Reaktion wurde mit 50 ml Xylol versetzt und die organische Phase abgetrennt. Durch Destillation erhielt man 18,1 g 2-Cyano-4'-methyl-biphenyl (Sdp. 140°C/mbar).

### Anwendungsbeispiel 5

15 g 2-Chlorbenzonitril, 14,8 g p-Toluolboronsäure und 28,9 g Natriumcarbonat wurden in 40 ml Glykol und 10 ml Wasser auf 120°C erhitzt. Bei 80°C gab man 0,1 Mol% einer nach Beispiel 3 hergestellten Katalysatorlösung zu. Nach beendeter Reaktion wurde mit 50 ml Xylol versetzt und die organische Phase abgetrennt. Durch Destillation erhielt man 19 g 2-Cyano-4'-methyl-biphenyl (Sdp. 140°C/mbar).

### Anwendungsbeispiel 6

15 g 2-Chlorbenzonitril, 14,8 g p-Toluolboronsäure und 12 g Natriumcarbonat wurden in 40 ml Glykol und 10 ml Wasser auf 120°C erhitzt. Bei 80°C gab man 0,1 Mol% einer nach Beispiel 1 hergestellten Katalysatorlösung zu. Nach beendeter Reaktion wurde mit 50 ml Xylol versetzt und die organische Phase abgetrennt. Durch Destillation erhielt man 18,5 g 2-Cyano-4'-methyl-biphenyl (Sdp. 140°C/mbar).

### Anwendungsbeispiel 7

15 g 2-Chlorbenzonitril, 14,8 g p-Toluolboronsäure und 12 g Natriumcarbonat wurden in 40 ml Glykol und 10 ml Wasser auf 120°C erhitzt. Bei 80°C gab man 0,1 Mol% einer nach Beispiel 3 hergestellten Katalysatorlösung zu. Nach beendeter Reaktion wurde mit 50 ml Xylol versetzt und die organische Phase abgetrennt. Durch Destillation erhielt man 18,7 g 2-Cyano-4'-methyl-biphenyl (Sdp. 140°C/mbar).

### Anwendungsbeispiel 8

15 g 2-Chlorbenzonitril, 14,8 g p-Toluolboronsäure und 12 g Natriumcarbonat wurden in 40 ml Glykol und 10 ml Wasser auf 120°C erhitzt. Bei 80°C gab man 0,1 Mol% einer nach Beispiel 2 hergestellten Katalysatorlösung zu. Nach beendeter Reaktion wurde mit 50 ml Xylol versetzt und die organische Phase abgetrennt. Durch Destillation erhielt man 18,1 g 2-Cyano-4'-methyl-biphenyl (Sdp. 140°C/mbar).

### Anwendungsbeispiel 9

15 g 2-Chlorbenzonitril, 14,8 g p-Toluolboronsäure und 28,9 g Natriumcarbonat wurden mit 50 ml p-Xylol, 40 ml Glykol und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 18,9 g 2-Cyano-4'-methylbiphenyl.

### Anwendungsbeispiel 10

15 g 2-Chlorbenzonitril, 14,8 g p-Toluolboronsäure und 28,9 g Natriumcarbonat wurden mit 50 ml p-Xylol 40 ml Glycerin und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 mi Wasser gewaschen und anschließend über Natriumsulfat getrocknet. Kristallisation aus n-Heptan ergab 18,5 g 2-Cyano-4'-methylbiphenyl.

### Anwendungsbeispiel 11

15 g 2-Chlorbenzonitril, 14,8 g p-Toluolboronsäure und 28,9 g Natriumcarbonat wurden mit 50 ml p-Xylol, 40 ml Triethylenglykol und 10 ml Wasser auf 120°C erhitzt. Bei 80°C gab man eine Mischung aus 24,7 mg Palladiumacetat und 0,55 ml TPP1 S/H₂O-Lösung (0,6 Molar) in 2,5 ml DMSO zu.

Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,4 g 2-Cyano-4'-methylbiphenyl.

### Anwendungsbeispiel 12

15 g 2-Chlorbenzonitril, 14,8 g p-Toluolboronsäure und 28,9 g Natriumcarbonat wurden mit 50 ml p-Xylol, 40 ml Diethylenglykol und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 18,2 g 2-Cyano-4'-methylbiphenyl.

### Anwendungsbeispiel 13

15 g 2-Chlorbenzonitril, 14,8 g p-Toluolboronsäure und 28,9 g Natriumcarbonat wurden mit 50 ml p-Xylol, 40 ml Diethanolamin und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,5 g 2-Cyano-4'-methylbiphenyl.

### Anwendungsbeispiel 14

15 g 2-Chlorbenzonitril, 14,8 g p-Toluolboronsäure und 28,9 g Natriumcarbonat wurden mit 50 ml p-Xylol, 40 ml Triethanolamin und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,8 g 2-Cyano-4'-methylbiphenyl.

### Anwendungsbeispiel 15

15 g 2-Chlorbenzonitril, 15,8 g p-Toluolboronsäure und 15,8 g Kaliumfluorid wurden mit 50 ml p-Xylol, 40 ml Glycerin und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 18,2 g 2-Cyano-4'-methylbiphenyl.

### Anwendungsbeispiel 16

15 g 2-Chlorbenzonitril, 15,8 g p-Toluolboronsäure und 15,8 g Kaliumfluorid wurden mit 50 ml p-Xylol, 40 ml Glykol und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 18,7 g 2-Cyano-4'-methylbiphenyl.

### Anwendungsbeispiel 17

15 g 2-Chlorbenzonitril, 14,8 g p-Toluolboronsäure und 15,8 g Kaliumfluorid werden mit 50 ml p-Xylol, 40 ml Diethylenglykol und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,8 g 2-Cyano-4'-methylbiphenyl.

### Anwendungsbeispiel 18

15 g 2-Chlorbenzonitril, 14,8 g p-Toluolboronsäure und 15,8 g Kaliumfluorid wurden mit 50 ml p-Xylol, 40 ml Triethylenglykol und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,2 g 2-Cyano-4'-methylbiphenyl.

### Anwendungsbeispiel 19

15 g 2-Chlorbenzonitril, 14,8 g p-Toluolboronsäure und 15,8 g Kaliumfluorid wurden mit 50 ml p-Xylol, 40 ml Diethanolamin und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 16,9 g 2-Cyano-4'-methylbiphenyl.

### Anwendungsbeispiel 20

15 g Chlorbenzonitril, 14,8 g p-Toluolboronsäure und 15,8 g Kaliumfluorid wurden mit 50 ml p-Xylol, 40 ml Triethanolamin und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,2 g 2-Cyano-4'-methylbiphenyl.

### Anwendungsbeispiel 21

15 g 2-Chlorbenzonitril, 14,8 g p-Toluolboronsäure und 28,9 g Natriumcarbonat wurden mit 50 ml p-Xylol, 40 ml Glykol und 10 ml Wasser auf 120°C erhitzt. Bei 80°C gab man eine Mischung aus 38,66 mg Palladium-ll-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 18,5 g 2-Cyano-4'-methylbiphenyl.

### Anwendungsbeispiel 22

15 g 2-Chlorbenzonitril, 14,8 g p-Toluolboronsäure und 28,9 g Natriumcarbonat wurden mit 50 ml p-Xylol, 40 ml Glycerin und 10 ml Wasser auf 120°C erhitzt. Bei 80"C gab man eine Mischung aus 38,66 mg Palladium-ll-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat
getrocknet. Kristallisation aus n-Heptan ergab 18,2 g 2-Cyano-4'-methylbiphenyl.

### Anwendungsbeispiel 23

15 g 2-Chlorbenzonitril, 14,8 g p-Toluolboronsäure und 28,9 g Natriumcarbonat wurden mit 50 ml p-Xylol, 40 ml Triethylenglykol und 10 ml Wasser auf 120°C erhitzt. Bei 80°C gab man eine Mischung aus 38,66 mg Palladium-ll-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,4 g 2-Cyano-4'-methylbiphenyl.

### Anwendungsbeispiel 24

15 g 2-Chlorbenzonitril, 14,8 g p-Toluolboronsäure und 28,9 g Natriumcarbonat wurden mit 50 ml p-Xylol, 40 ml Diethylenglykol und 10 ml Wasser auf 120°C erhitzt. Bei 80°C gab man eine Mischung aus 38,66 mg Palladium-ll-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,1 g 2-Cyano-4'-methylbiphenyl.

### Anwendungsbeispiel 25

15 g 2-Chlorbenzonitril, 14,8 g p-Toluolboronsäure und 28,9 g Natriumcarbonat wurden mit 50 ml p-Xylol, 40 ml Diethanolamin und 10 ml Wasser auf 120°C erhitzt. Bei 80°C gab man eine Mischung aus 38,66 mg Palladium-ll-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,5 g 2-Cyano-4'-methylbiphenyl.

### Anwendungsbeispiel 26

15 g 2-Chlorbenzonitril, 14,8 g p-Toluolboronsäure und 28,9 g Natriumcarbonat wurden mit 50 ml p-Xylol, 40 ml Triethanolamin und 10 ml Wasser auf 120°C erhitzt. Bei 80°C gab man eine Mischung aus 38,66 mg Palladium-ll-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,8 g 2-Cyano-4'-methylbiphenyl.

### Anwendungsbeispiel 27

15 g 2-Chlorbenzonitril, 14,8 g p-Toluolboronsäure und 15,8 g Kaliumfluorid wurden mit 50 ml p-Xylol, 40 ml Glycerin und 10 ml Wasser auf 120°C erhitzt. Bei 80°C gab man eine Mischung aus 38,66 mg Palladium-ll-chlorid und 1,1 ml TPPTS/H₂O-Lösung (0,6 Molar) in 2,5 ml DMSO zu.
Nach beendeter Reaktion werden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 18,9 g 2-Cyano-4'-methylbiphenyl.

### Anwendungsbeispiel 28

15 g 2-Chlorbenzonitril, 14,8 g p-Toluolboronsäure und 15,8 g Kaliumfluorid wurden mit 50 ml p-Xylol, 40 ml Glykol und 10 ml Wasser auf 120°C erhitzt. Bei 80°C gab man eine Mischung aus 38,66 mg Palladium-ll-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 18,8 g 2-Cyano-4'-methylbiphenyl.

### Anwendungsbeispiel 29

15 g 2-Chlorbenzonitril, 14,8 g p-Toluolboronsäure und 15,8 g Kaliumfluorid wurden mit 50 ml p-Xylol, 40 ml Diethylenglykol und 10 ml Wasser auf 120°C erhitzt. Bei 80°C gab man eine Mischung aus 38,66 mg Palladium-II-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 18,0 g 2-Cyano-4'-methylbiphenyl.

### Anwendungsbeispiel 30

15 g 2-Chlorbenzonitril, 14,8 g p-Toluolboronsäure und 15,8 g Kaliumfluorid wurden mit 50 ml p-Xylol, 40 ml Triethylenglykol und 10 ml Wasser auf 120°C erhitzt. Bei 80°C gab man eine Mischung aus 38,66 mg Palladium-ll-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 16,9 g 2-Cyano-4'-methylbiphenyl.

### Anwendungsbeispiel 31

15 g 2-Chlorbenzonitril, 14,8 g p-Toluolboronsäure und 15,8 g Kaliumfluorid wurden mit 50 ml p-Xylol, 40 ml Diethanolamin und 10 ml Wasser auf 120°C erhitzt. Bei 80°C gab man eine Mischung aus 38,66 mg Palladium-ll-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasenwurden mit 20 ml Wasser gewaschen und anschließend über Natriumsulfat getrocknet. Kristallisation aus n-Heptan ergab 17,1 g 2-Cyano-4'-methylbiphenyl.

### Anwendungsbeispiel 32

15 g 2-Chlorbenzonitril, 14,8 g p-Toluolboronsäure und 15,8 g Kalumfluorid wurden mit 50 ml p-Xylol, 40 ml Triethanolamin und 10 ml Wasser auf 120°C erhitzt. Bei 80°C gab man eine Mischung 38,66 mg Palladium-ll-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 18,0 g 2-Cyano-4'-methylbiphenyl.

### Anwendungsbeispiel 33

15 g 2-Chlorbenzonitril, 14,8 g p-Toluolboronsäure und 28,9 g Natriumcarbonat wurden mit 50 ml p-Xylol, 40 ml Glykol und 10 ml Wasser auf 120°C erhitzt. Bei 80°C gab man 19,3 mg Palladiumchlorid, 17,9 mg Natriumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 mi Toluol gewaschen. Die vereinigten organischen Phasen wurden mit 20 ml Wasser gewaschen und anschließend über Natriumsulfat getrocknet. Kristallisation aus n-Heptan ergab 18,7 g 2-Cyano-4'-methylbiphenyl.

### Anwendungsbeispiel 34

15 g 2-Chlorbenzonitril, 14,8 g p-Toluolboronsäure und 28,9 g Natriumcarbonat werden mit 50 ml p-Xylol, 40 ml Glycerin und 10 ml Wasser auf 120°C erhitzt. Bei 80°C gab man eine Mischung aus 19,3 mg Palladiumchlorid, 17,9 mg Natriumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 18,3 g 2-Cyano-4'-methylbiphenyl.

### Anwendungsbeispiel 35

15 g 2-Chlorbenzonitril, 14,8 g p-Toluolboronsäure und 28,9 g Natriumcarbonat wurden mit 50 ml p-Xylol, 40 ml Triethylenglykol und 10 ml Wasser auf 120°C erhitzt. Bei 80°C gab man eine Mischung aus 19,3 mg Palladiumchlorid,
17,9 mg Natriumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,4 g 2-Cyano-4'-methylbiphenyl.

### Anwendungsbeispiel 36

15g 2-Chlorbenzonitril, 14,8 g p-Toluolboronsäure und 28,9 g Natriumcarbonat wurden mit 50 ml p-Xylol, 40 ml Diethylenglykol und 10 ml Wasser auf 120°C erhitzt. Bei 80°C gab man eine Mischung aus 19,3 mg Palladiumchlorid,
17,9 mg Natriumacetat und 0,55 ml TPPTS/H₂O-Lösung (0,6 Molar) in 2,5 ml DMSO zu.

Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 18,3 g 2-Cyano-4'-methylbiphenyl.

### Anwendungsbeispiel 37

15 g 2-Chlorbenzonitril, 14,8 g p-Toluolboronsäure und 28,9 g Natriumcarbonat wurden mit 50 ml p-Xylol, 40 ml Diethanolamin und 10 ml Wasser auf 120°C erhitzt. Bei 80°C gab man eine Mischung aus 19,3 mg Palladiumchlorid,
17,9 mg Natriumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,5 g 2-Cyano-4'-methylbiphenyl.

### Anwendungsbeispiel 38

15 g 2-Chlorbenzonitril, 14,8 g p-Toluolboronsäure und 28,9 g Natriumcarbonat wurden mit 50 ml p-Xylol, 40 ml Triethanolamin und 10 ml Wasser auf 120°C erhitzt. Bei 80°C gab man 19,3 mg Palladiumchlorid, 17,9 mg Natriumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,8 g 2-Cyano-4'-methylbiphenyl.

### Anwendungsbeispiel 39

15 g 2-Chlorbenzonitril, 14,8 g p-Toluolboronsäure und 28,9 g Natriumcarbonat wurden mit 50 ml p-Xylol, 40 ml DMSO und 10 ml Wasser auf 120°C erhitzt. Bei 80°C gab man eine Mischung aus 24,7 mg Palladiumacetat (II) und 0,55 ml TPPTS/H₂O (0,6 Molar) in 2,5 ml DMSO zu.

Nach beender Reaktion wurden die Phasen getrennt. Die wäßrige Phase wurde mit 50 ml Xylol gewaschen. Die vereinigten organischen Phasen wurden mit 20 ml Wasser gewaschen und anschließend über Natriumsulfat getrocknet. Das Lösungsmittel wurde eingedampft und der Rückstand aus n-Heptan kristallisiert. Ausbeute: 18,6 g (88 % d.Th.) 2-Cyano-4'-methylbiphenyl.

### Anwendungsbeispiel 40

15 g 2-Chlorbenzonitril, 14,8 g p-Toluolboronsäure und 28,9 g Natriumcarbonat wurden mit 50 ml p-Xylol, 40 ml DMSO und 10 ml Wasser auf 120°C erhitzt. Bei 80°C ergab man eine Mischung aus 38,66 mg Palladium-ll-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Xylol gewaschen. Die vereinigten organischen Phasen wurden mit 20 ml Wasser gewaschen und anschließend über Natriumsulfat getrocknet. Das Lösungsmittel wurde eingedampft und der Rückstand aus n-Heptan kristallisiert. Ausbeute: 18,2 g (86 % d.th.) 2-Cyano-4'-methylbiphenyl.

### Anwendungsbeispiel 41

15 g 2-Chlorbenzonitril, 14,8 g p-Toluolboronsäure und 28,9 g Natriumcarbonat wurden mit 50 ml p-Xylol, 40 ml DMSO und 10 ml Wasser auf 120°C erhitzt. Bei 80°C gab man eine Mischung aus 19,3 mg Palladium-ll-chlorid, 17,9 mg Natriumacetat und 0,55 ml TPPTS/H₂O-Lösung (0,6 Molar) in 2,5 ml DMSO zu. Nach beendeter Reaktion wurden die Phasen getrennt. Die wäßrige Phase wird mit 50 ml Xylol gewaschen. Die vereinigten organischen Phasen wurden mit 20 ml Wasser gewaschen und anschließend über Natriumsulfat getrocknet. Das Lösungsmittel wurde eingedampft und der Rückstand aus n-Heptan kristallisiert. Ausbeute: 18,8 g (89 % d.Th.) 2-Cyano-4'-methylbiphenyl.

### Anwendungsbeispiel 42

15 g 2-Chlorbenzonitril, 14,8 g p-Toluolboronsäure und 28,9 g Natriumcarbonat wurden mit 50 ml p-Xylol und 40 ml DMSO auf 120°C erhitzt. Bei 80°C gab man eine Mischung aus 24,7 mg Palladiumacetat (II) und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Xylol gewaschen. Die vereinigten organischen Phasen wurden mit 20 ml Wasser gewaschen und anschließend über Natriumsulfat getrocknet. Das Lösungsmittel wurde eingedampft und der Rückstand aus n-Heptan kristallisisert. Ausbeute: 18,0 g (85 % d.Th.) 2-Cyano-4'-methylbiphenyl.

### Anwendungsbeispiel 43

### Kreuzkupplung von 2-Chlorbenzonitril mit 4-Toluolboronsäure

Zur Herstellung des Katalysators werden 38,8 mg (0,219 mmol) Palladium-(II)-chlorid und 54,0 mg (0,657 mmol) Natriumacetat in 2,4 ml DMSO unter Argonatmosphäre 30 min bei 23°C gerührt. Anschließend werden 1,99 ml (0,875 mmol) einer 0,44 molaren wäßrigen Lösung von Natrium-4-diphenylphosphinophenyl-phosphinat, hergestellt wie unten angegeben zugegeben und die Suspension wird weitere 30 min bei 23°C gerührt.
Unter Argonatmosphäre werden 30,0 g (0,2181 mol) 2-Chlorbenzonitril, 32,6 g (0,240 mol) 4-Toluolboronsäure und 16,2 g (70 mol%) Natriumcarbonat in 120 ml Ethylenglycol verrührt. Man gibt 20 ml Wasser zu und erwärmt auf 80°C. Nun wird die oben beschriebene Katalysator-Suspension zugegeben und man erhitzt 5 Stunden unter Rückfluß.
Bei 23°C wird die Mischung mit 100 ml Essigester versetzt. Die organische Phase wird abgetrennt, am Rotationsverdampfer eingeengt und im Vakuum fraktioniert destilliert. Man erhält 31,6 g (75 % d. Th.) 2-Cyano-4'-methyl-biphenyl (Kp. 140°C/1,0 mbar; Fp. 50°C).

## Patentansprüche

1. Katalysatorsystem, insbesondere zur Durchführung von Kreuzkupplungsreaktionen, erhältlich durch Umsetzung
a) einer Palladium(ll)verbindung mit
b) einem wasserlöslichen Phosphanliganden und
c) einem Sulfoxid oder mehrwertigem wasserlöslichen Alkohol;
aus der Gruppe Glykole, Glycerin, Oligoglyceride, die auch teilverestert sein können, Di-, Tri- und Tetraethylenglykol oder auch Polyethylenglykole der allgemeinen Formel (VIII), 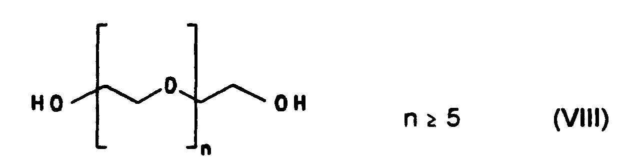 mehrwertige Alkan- oder Alkenole, mehrwertige Cycloalkanole, mehrwertige Arylgruppen enthaltende Alkanole, mehrwertige Aminoalkohole, mehrwertige Iminoalkohole oder wie 1,1',1"-Nitrilo-tri-2-propanol, 1,3,5-Tri-(2-hydroxyethyl)-isocyanursäure und Dihydroxyaceton.

2. Katalysatorsystem nach Anspruch 1, **dadurch gekennzeichnet, daß** ein Sulfoxid der Formel (IX) eingesetzt wird: 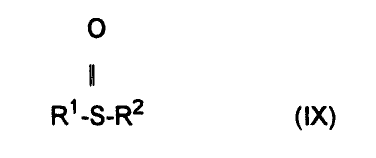 wobei R¹, R² aliphatische oder aromatische Kohlenwasserstoffe sind, die gegebenenfalls substituiert oder miteinander verknüpft sein können.

3. Katalysatorsystem nach Anspruch 1 oder 2 **dadurch gekennzeichnet, daß** die wasserlöslichen Phosphanliganden aus der Gruppe mit Carboxylat- bzw. Carbonsäure, Ammonium, Phosphonium, Sulfonat- bzw. Sulfonsäure, Phosphonat- bzw. Phosphonsäure, Polyalkohole mit geeigneter Anzahl von Hydroxyfunktionen versehene Gruppen, Polyalkylenglykole mit geeigneter Kettenlänge, versehene Tri-n-alkylphosphane, Triarylphosphane, Dialkylarylphosphane, Alkyldiarylphosphane und Heteroarylphosphane wobei die drei Substituenten am Phosphor gleich oder verschieden, chiral oder achiral sein können und wobei einer oder mehrere der Substituenten die Phosphorgruppen mehrerer Phosphane verknüpfen können und wobei ein Teil dieser Verknüpfung auch ein oder mehrere Metallatome sein können.

4. Katalysatorsystem nach einem oder mehreren der vorhergehenden Ansprüche, **dadurch gekennzeichnet, daß** man eine Palladium(ll)verbindung aus der Gruppe Palladium(ll)salze, Palladiumtetrachlorosäure oder deren Salze einsetzt.

5. Verfahren zur Herstellung eines Katalysatorsystems nach einem oder mehrenen der Ansprüche 1 bis 4, **dadurch gekennzeichnet, daß** man
a) eine Palladium(ll)verbindung mit
b) einem wasserlöslichen Phosphanliganden und
c) einem Sulfoxid oder mehrwertigem Alkohol
umsetzt.

6. Verfahren nach Anspruch 5, **dadurch gekennzeichnet, daß** man der Reaktion Wasser zusetzt.

7. Verfahren nach Anspruch 5 oder 6, **dadurch gekennzeichnet, daß** man einen Lösungsvermittler für die Palladiumverbindung zusetzt.

8. Verfahren nach Anspruch 7, **dadurch gekennzeichnet, daß** der Lösungsvermittler Natriumacetat ist.

9. Verwendung eines Katalysatorsystems, erhältlich durch Umsetzung
a) einer Palladium(ll)verbindung mit
b) einem wasserlöslichen Phosphanliganden und
c) einem Sulfoxid oder mehrwertigem Alkohol,
zur Durchführung von C-C-Verknüpfungsreaktionen.

## Claims

1. A catalyst system, in particular for carrying out cross-coupling reactions, obtainable by reacting
a) a palladium(II) compound with
b) a water-soluble phosphine ligand and
c) a sulfoxide or polyhydric water-soluble alcohol; selected from the group consisting of glycols, glycerol, oligoglycerides, which may also be partially esterified, diethylene, triethylene and tetraethylene glycols or polyethylene glycols of the formula (VIII), 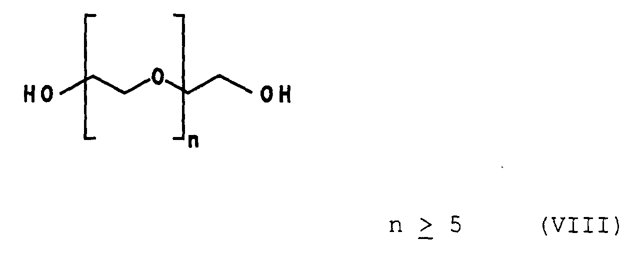 polyhydric alkanols or alkenols, polyhydric cycloalkanols, polyhydric alkanols containing aryl groups, polyhydric aminoalcohols, polyhydric iminoalcohols or 1,1',1"-nitrilotri-2-propanol, 1,3,5-tri(2-hydroxyethyl)isocyanuric acid and dihydroxyacetone.

2. A catalyst system as claimed in claim 1, wherein the sulfoxide used has the formula (IX) 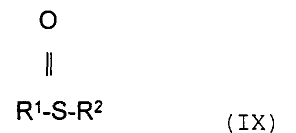 where R¹, R² are aliphatic or aromatic hydrocarbons which may, if desired, be substituted or linked to one another.

3. A catalyst system as claimed in claim 1 or 2, wherein the water-soluble phosphine ligands are selected from the group consisting of tri-n-alkylphosphines, triarylphosphines, dialkylarylphosphines, alkyldiarylphosphines and heteroarylphosphines provided with carboxylate or carboxylic acid, ammonium, phosphonium, sulfonate or sulfonic acid, phosphonate or phosphonic acid groups or with polyalcohols having a suitable number of hydroxy functions or polyalkylene glycols having a suitable chain length, where the three substituents on the phosphorus can be identical or different and chiral or achiral and one or more of the substituents can link the phosphorus groups of a plurality of phosphines and part of this linkage can also be one or more metal atoms.

4. A catalyst system as claimed in one or more of the preceding claims, wherein the palladium(II) compound used is selected from the group consisting of palladium(II) salts, tetrachloropalladic acid or salts thereof.

5. A process for preparing a catalyst system as claimed in one or more of claims 1 to 4, which comprises reacting
a) a palladium(II) compound with
b) a water-soluble phosphine ligand and
c) a sulfoxide or polyhydric alcohol.

6. The process as claimed in claim 5, wherein water is added to the reaction.

7. The process as claimed in claim 5 or 6, wherein a solubilizer for the palladium compound is added.

8. The process as claimed in claim 7, wherein the solubilizer is sodium acetate.

9. The use of a catalyst system obtainable by reacting
a) a palladium(II) compound with
b) a water-soluble phosphine ligand and
c) a sulfoxide or polyhydric alcohol
for carrying out C-C-linkage reactions.

## Revendications

1. Système catalytique, en particulier pour la mise en oeuvre de réactions de copulation croisée, que l'on peut obtenir par réaction
a) d'un composé de palladium (II) avec
b) d'un ligand phosphane hydrosoluble et
c) d'un sulfoxyde ou alcool multifonctionnel hydrosoluble ;
pris dans le groupe comportant des glycols, la glycérine, des oligoglycérides qui peuvent également être partiellement estérifiés, des di-, tri- et tétraéthylèneglycols ou aussi des polyéthylèneglycols de formule générale (VIII) 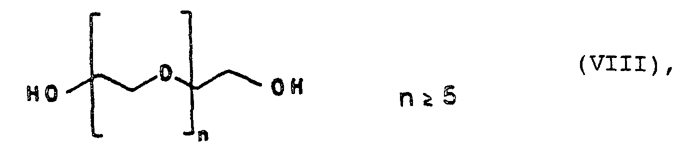 des alcanols ou alcénols multifonctionnels, des cycloalcanols multifonctionnels, des alcanols multifonctionnels contenant des groupes aryle, des aminoalcools multifonctionnels, des iminoalcools multifonctionnels ou tels que le 1,1',1"-nitrilo-tri-2-propanol, l'acide 1,3,5-tri-(2-hydroxyéthyl)isocyanurique et la dihydroxyacétone.

2. Système catalytique selon la revendication 1, **caractérisé en ce qu'**on utilise un sulfoxyde de formule (IX) : 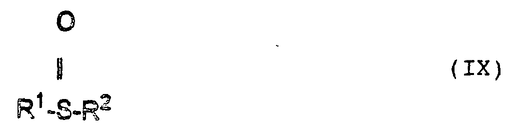 R¹, R² étant des hydrocarbures aliphatiques ou aromatiques, qui sont éventuellement substitués ou peuvent être reliés entre eux.

3. Système catalytique selon la revendication 1 ou 2, **caractérisé en ce que** les ligands phosphanes hydrosolubles sont pris dans le groupe comportant des tri-n-alkylphosphanes, des triarylphosphanes, des dialkylarylphosphanes, des alkyldiarylphosphanes et des hétéroarylphosphanes munis de groupes carboxylate ou acide carboxylique, ammonium, phosphonium, sulfonate ou acide sulfonique, phosphonate ou acide phosphonique, polyalcools présentant le nombre approprié de fonctions hydroxy, polyalkylèneglycols ayant une longueur de chaîne appropriée, les trois derniers substituants sur l'atome de phosphore peuvent être identiques ou différents, chiraux ou achiraux et l'un ou plusieurs des substituants pouvant relier les groupes phosphore de plusieurs phosphanes et une partie de ces liaisons pouvant être un ou plusieurs atomes métalliques également.

4. Système catalytique selon une ou plusieurs des revendications précédentes, **caractérisé en ce qu'**on utilise un composé de palladium(II) pris dans le groupe des sels de palladium(II), d'acide tétrachloropalladique ou leurs sels.

5. Procédé pour la préparation d'un système catalytique selon une ou plusieurs des revendications 1 à 4, **caractérisé en ce qu'**on fait réagir
a) un composé de palladium(II) avec
b) un ligands phosphane hydrosoluble et
c) un sulfoxyde ou alcool multifonctionnel.

6. Procédé selon la revendication 5, **caractérisé en ce qu'**on ajoute de l'eau à la réaction.

7. Procédé selon la revendication 5 ou 6, **caractérisé en ce qu'**on ajoute un solubilisant pour le composé de palladium.

8. Procédé selon la revendication 7, **caractérisé en ce que** le solubilisant est l'acétate de sodium.

9. Utilisation d'un système catalytique que l'on peut obtenir par réaction
a) d'un composé de palladium(II) avec
b) un ligands phosphane hydrosoluble et
c) un sulfoxyde ou alcool multifonctionnel,
pour la mise en oeuvre de réactions de liaison C-C.
